# EUROPEAN PATENT APPLICATION

(11) **EP 4 080 211 A1**
(43) Date of publication of application: **26.10.2022**
(21) Application number: 20901006.5
(22) Date of filing: 19.02.2020
(51) Int. Cl.: G01N 33/543, G01N 35/00, G01N 35/04

(54) **AUTOMATED ANALYSIS DEVICE FOR LIQUID-PHASE IMMUNOASSAY, AND IMMUNOASSAY METHOD USING SAME**

(30) Priority: 18.12.2019 KR 20190169600
(71) Applicant: Boditech Med Inc., Chuncheon-si, Gangwon-do 24398 (KR)
(72) Inventor: JOO, Hoo Don, Chuncheon-si Gangwon-do 24449 (KR); KIM, Hyung Hoon, Chuncheon-si Gangwon-do 24399 (KR); KIM, Hak Seong, Namyangju-si Gyeonggi-do 12251 (KR); GIL, Hye Su, Chuncheon-si Gangwon-do 24253 (KR); SAYYED, Danishmalik Rafiq, Chuncheon-si Gangwon-do 24311 (KR)
(74) Representative: De Vries & Metman
(86) International application number: PCT/KR2020/002410
(87) International publication number: WO 2021/125440

(57) **Abstract**

The present invention relates to an automated immunoassay device for detecting particular ingredients contained in a biological sample, and a method therefor.

## Description

### CROSS REFERENCE TO RELATED APPLICATION

The present application claims priority to Korean Patent Application No. 10-2019-0169600, filed December 18, 2019, the entire contents of which is incorporated herein for all purposes by this reference.

### BACKGROUND OF THE INVENTION

### Field of the Invention

The present invention relates to an automated liquid immunoassay device and method therefor.

### Description of the Related Art

As medicine, biotechnology and various related technologies develop, the inspections have been widely performed to detect various molecular indicators such as blood cells, genes, proteins, antigens, pathogens, etc. in predetermined biological samples such as urine and blood. The inspection process generally comprises the steps of: taking a sample, reacting the sample with a predetermined reagent suitable for the desired indicator, and observing and analyzing the changes that occur. In this way, the various molecular indicators included in a sample can be analyzed qualitatively and/or quantitatively, so that the information on diagnosis, progress or prognosis of disease can be obtained.

One of the techniques widely used in this inspection process is an immunoassay technique called EIA (Enzyme ImmunoAssay) based on specific binding between antigens and antibodies. This includes color change measurement method (chromogenic or colorimetric) to measure the color reaction by absorbance, chemiluminescence method and fluorescence method, depending on the type of substrate used for detection of the analyte. It also includes a sandwich-type immunoassay or competition-type immunoassay, also called Enzyme Linked Immunosorbent Assay, depending on the assay.

In such assays, removal of non-specific reactants is desirable for high sensitivity detection of high specificity regardless of how to use. That is, after the reaction between the reagent and the sample in the inspection process, it is required to purify the reaction product for accurate detection of the reaction product.

The most effective method for removing non-specific reactants is physical washing or purification. Among them, it is preferable to remove non-specific reactants by using a washing method using magnetic particles. This washing method using magnetic particles requires a separate device for separating and washing, and non-specific reactants can be removed by repeating scattering and collecting magnetic particles. However, the method of repeating scattering and collecting magnetic particles has a disadvantage since it requires such long washing process that many magnetic beads may be lost. So, it is necessary to additionally develop a test device and method for removing non-specific reactants effectively within a short time without losing magnetic beads.

### SUMMARY OF THE INVENTION

The present invention provides a washing device and a washing method for purifying the reaction product in the automated liquid immunoassay method.

The present invention for achieving the above-mentioned objectives provides an immunoassay device using magnetic beads, comprises: a straw arm capable of fixing a washing tip to a lower part and having a hollow penetrating vertically upward and downward inside; a magnetic beam positioned in a hollow of the straw arm and capable of moving vertically upward and downward; a movable body to which the straw arm is fixed; a movable body drive unit for moving the movable body; a driving motor for moving the magnetic beam; and a control unit for controlling the movable body drive unit.

Preferably, the device further comprises: a reaction chamber including magnetic beads to which biological samples and reagents are attached; a washing chambers where the non-specific biological samples are washed and scattered in the reagents; and a detection chamber where magnetic beads which the non-specific biological samples are attached to in the reagents are scattered.

The present invention provides an immunoassay method using magnetic beads, including steps of: bonding analytes in biological samples and magnetic beads through an antigen-antibody reaction by injecting the biological samples into a first vessel that contains the magnetic beads; positioning a magnetic beam in a hollow portion of the washing tip; attaching the magnetic beads to a surface of a washing tip by inserting the washing tip in which the magnetic beam is positioned into the first vessel; moving the washing tip which the magnetic beads is attached to a second vessel; physically washing non-specific reactants from a surface of the washing tip to which the magnetic beads are attached in the second chamber; moving the washing tip from which the non-specific reactant has been removed to the detection chamber; and scattering the magnetic beads on the surface of the washing tip in the detection chamber by raising the magnetic beam positioned in the hollow portion of the washing tip.

Preferably, the step physically washing the samples includes a step of: moving vertically upward and downward the washing tip repeatedly which the magnetic beam is inserted into.

The present invention does not repeat scattering and collecting magnetic beads, removes non-specific bonding by repeatedly moving vertically upward and downward when they are collected, and reduces the loss and the missing of magnetic beads that may occur in the process of repeating scattering and collection so as to enhance the reproducibility of the measurement result and reduce the coefficient of variation (CV). In addition, owing to the simplification of the washing process, the measurement time is reduced so that the number of measurements per hour (throughput) can be increased and the performance and competitiveness of the equipment can be improved.

### BRIEF DESCRIPTION OF THE DRAWINGS

The above and other objects, features and other advantages of the present invention will be more clearly understood from the following detailed description when taken in conjunction with the accompanying drawings, in which:
FIG. 1 is a schematic diagram showing the process of sandwich-type immunoassay using magnetic beads used in the device according to an embodiment of the present invention.
FIG. 2 is a schematic diagram showing the process of competition-type immunoassay using magnetic beads used in the device according to an embodiment of the present invention.
FIG. 3 is a block diagram showing a schematic structure of a module in which a magnetic beam and a washing tip are combined in the automated liquid immunoassay device according to an embodiment of the present invention.
FIG. 4 is an enlarged view showing the area of the washing tip in detail in the automated liquid immunoassay device according to an embodiment of the present invention.
FIG. 5 shows one form of a reaction chamber, a washing chamber, and a detection chamber used in the device according to an embodiment of the present invention.
FIG. 6 is a flow chart of a washing method according to an embodiment of the present invention.

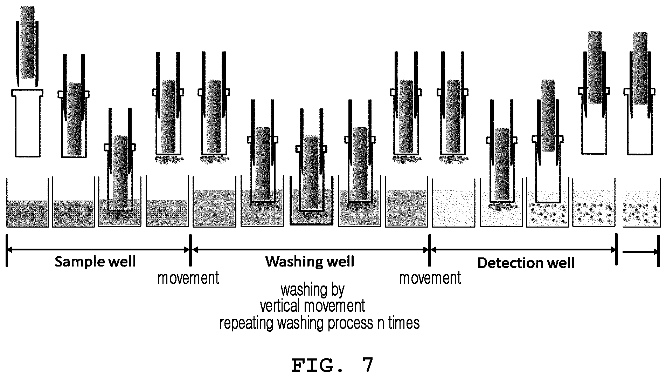

FIG. 7 is a schematic diagram showing a washing method in the automated liquid immunoassay method using magnetic beads according to another embodiment of the present invention.

### DETAILED DESCRIPTION OF THE INVENTION

Hereinafter, exemplary embodiments of the present invention will be described with reference to the accompanying drawings. These embodiments are illustrative and do not limit the present invention in any way.

Hereinafter, most of all, the terms used in the present specification and the principles of chemical reactions used with the device will be described.

In the present specification, "detection" is used to mean analyzing quantitatively or qualitatively the analyte contained in sample by purifying the reaction product after the reaction between the reagent and the sample in order to determine the presence or the amount of the analyte contained in the sample. The detection result is read by an automated liquid immunoassay device according to an embodiment of the present invention.

In this specification, the term "inspection" is used as a term encompassing all of detection, analysis and reading.

The term "sample" used in this specification refers to a composition that is expected to include an analyte, and a sample that can be used in the present invention is a liquid material or a fluid material similar to liquid. The sample used in an embodiment of the present invention is a biological sample, and can be a biological body-derived body component such as whole blood, plasma, serum, urine, saliva, manure and cell extracts.

The term "analyte" used in this specification refers to an analytical compound in a sample, also referred to as a target or indicator, including but not limited to a protein component such as an antigen and a nucleic acid material such as gene.

In this specification, "reagent" is substance used in admixture with a sample for quantitative or qualitative analysis of an analyte contained in a sample, and varies according to a specific analyte. It may include but is not limited to, for example, a reaction buffer, a dilution buffer, a detection buffer, a wash buffer or various substances in the sample such as enzymes, substrates or certain antibodies that react with antigens for instance.

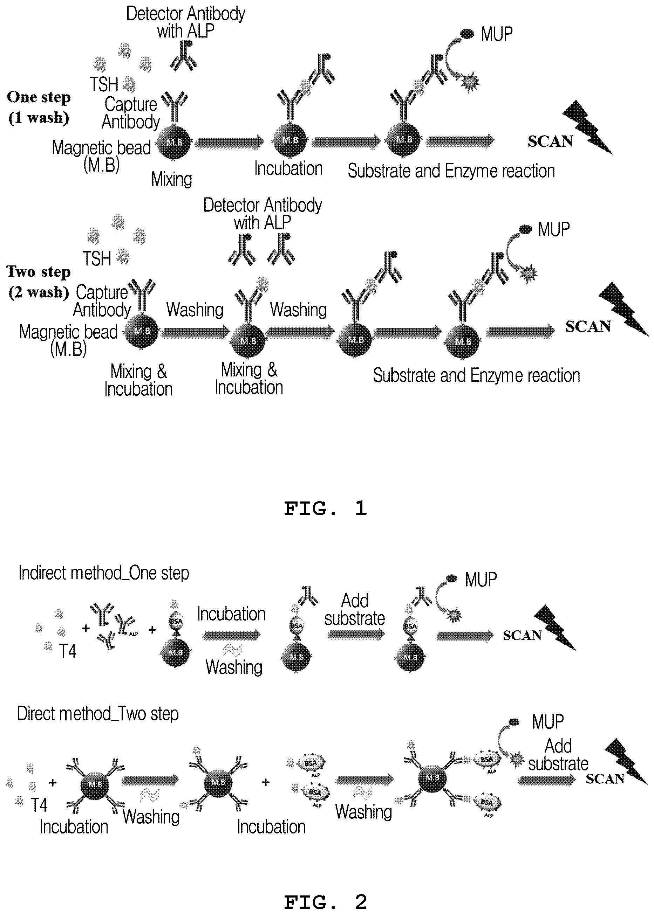

The immunoassay device according to an embodiment of the present invention relates to a device optimized for physical washing to separate unreacted substances from reaction products using magnetic beads before detection of analyte as well as for detection of analyte or specific components included in a biological sample through an Enzyme Linked Immunosorbent Assay (ELISA)-based reaction based on specific bonding between antigens and antibodies, for example, reactions as shown in FIGS. 1 and 2.

FIGS. 1 and 2 illustrate various ELISA analysis process for analyzing analyte. Sandwich immunoassay refers to a type of immunoassay in which a capture antibody and a detector antibody are bonded to each other in the form of sandwich, and the detector antibody is chemically bonded with an enzyme so as to induce a quantitative reaction with a substrate. At this time, used is the conjugate in which the capture antibody is chemically or physically bonded to the magnetic beads and the detector antibody is bonded to the enzyme. Depending on how many steps in which the washing is performed, the sandwich immunoassay using the magnetic beads can be categorized into two types: one-step assay and two-step assay. The two-step assay is performed as follows: the sample is first reacted with the capture antibody, washed, and then lastly reacted with the detector antibody. The one-step assay is as follows: the sample is reacted with the capture antibody and the detector antibody at the same time (FIG. 1) .

The competition assay which is widely used to detect small amounts of protein molecules besides the sandwich immunoassay is also categorized into two types: indirect competition assay and direct competition depending on whether competition proteins or antibodies are conjugated to the magnetic beads. And it can be also categorized into one-step assay and two-step assay according to how many steps the immunoassay is performed in. For example, FIG. 2 illustrates an example of indirect competition immunoassay and an example of direct competition immunoassay among competition assays.

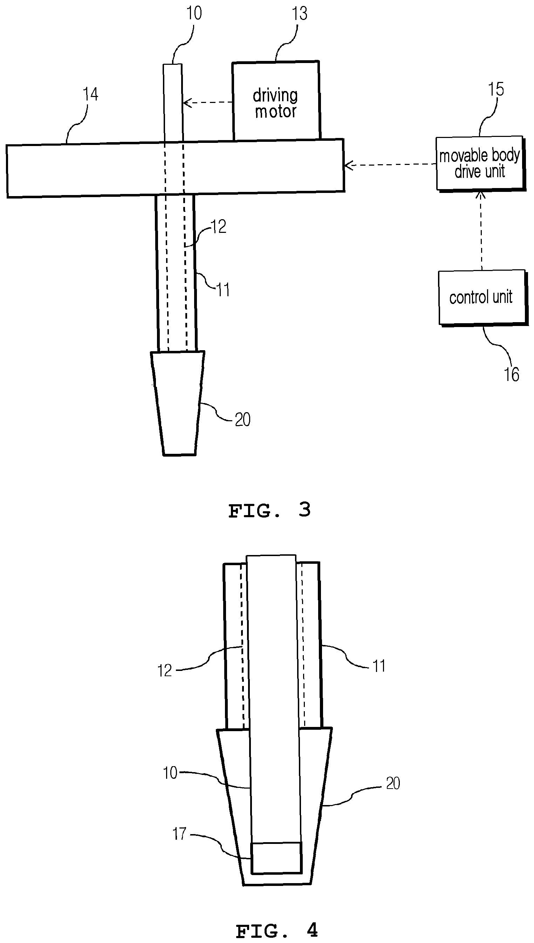

In an embodiment according to the present invention, referring to FIG. 3, an immunoassay device using magnetic beads, comprises: a straw arm 11 capable of fixing a washing tip 20 to a lower part and having a hollow 12 penetrating vertically upward and downward inside; a magnetic beam 10 positioned in a hollow 12 of the straw arm 11 and capable of moving vertically upward and downward; a movable body 14 to which the straw arm is fixed; a movable body drive unit 15 for moving the movable body horizontally; a driving motor 13 for moving the magnetic beam vertically upward and downward; and a control unit 16 for controlling the movable body drive unit.

Hereinafter, the operation of each component listed above will be described in detail.

The control unit 16 controls the movable body drive unit 15 to move the movable body 14 to a desired position.

A straw arm 11 is fixed to the movable body 14. So, the straw arm moves together when the movable body moves.

The straw arm 11 can be mounted by fixing the washing tip 20 to the lower part. The straw arm has a hollow 12 that penetrates vertically upward and downward inside. A magnetic beam 10 that can move vertically upward and downward is positioned in a hollow of the straw arm. A driving motor 13 is provided to move the magnetic beam vertically upward and downward. It is desirable to fix the drive motor 13 to the movable body so that the magnetic beam can move relative to the straw arm fixed to the movable body.

FIG. 4 is an enlarged view showing in detail the washing tip part in an automated liquid immunoassay device according to an embodiment of the present invention. It comprises: a magnetic beam 10 disposed in the hollow 12 of the straw arm 11; and a permanent magnet 17 attached to an end of the magnetic beam. When the magnetic beam is lowered by the driving motor 13, a permanent magnet may be disposed inside the washing tip 20 fitted to the straw arm 11.

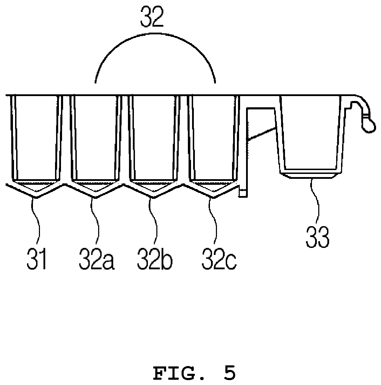

FIG. 5 shows a reaction chamber 31, a washing chamber 32, and a detection chamber 33 in the immunoassay device according to an embodiment of the present invention.

The device further comprises: a reaction chamber 31 including magnetic beads to which biological samples and reagents are attached; a washing chambers 32 where the non-specific biological samples are washed; and a detection chamber 33 for detecting the non-specific biological samples in the reagents.

The reaction chamber 31 includes the magnetic beads to which a biological sample and a reagent are attached. The analyte among the reagent on the surface of the magnetic beads and the biological samples undergoes antigen and antibody reactions. And, the analyte which is the reaction result, the bonded magnetic beads, and the non-specific biological samples remain in the reaction chamber.

The washing chamber 32 is a place where washing is performed in order to remove non-specific biological samples entangled in the reaction result in the reaction chamber. And, a plurality of washing chambers may be provided. Three washing chambers 32a, 32b and 32c are included in the embodiment of the present invention.

The reaction chamber 31 is a chamber for scattering and detecting the magnetic beads attached to the surface of the washing tip.

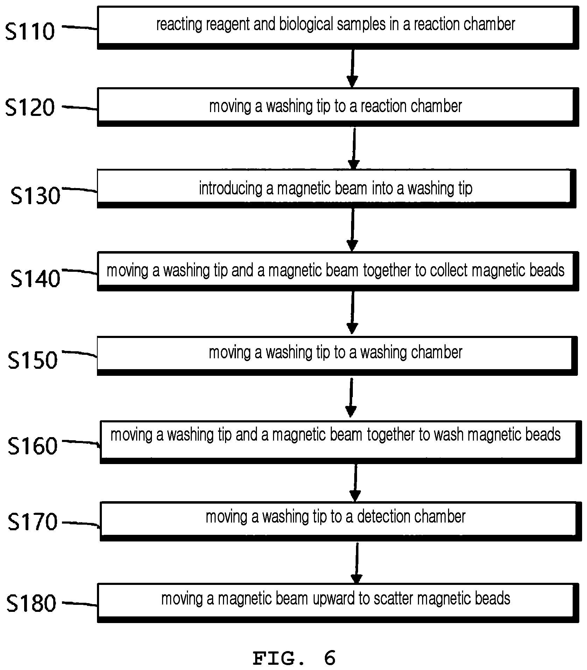

FIG. 6 is a flow chart of a magnetic bead washing method according to another embodiment of the present invention.

The magnetic beads which reagents are attached to is contained in the reaction chamber 31. The biological samples in which non-specific reactants and analytes are mixed are injected so as to cause them to react. (S110) The reagent of the magnetic beads and analytes in the biological samples can be bonded with each other through an antigen-antibody reaction. The washing tip 20 fitted to the straw arm 11 is moved to the reaction chamber 31. (S120) The magnetic beam 10 positioned in a hollow portion 12 of the straw arm 11 is lowered using the driving motor 13 to be inserted into the washing tip. (S130) The magnetic beads are collected onto a surface of the washing tip due to the magnetism of the magnetic beam In the washing tip. (S140) The washing tip which the magnetic beads are attached to the surface of is moved together with the magnetic beam to a washing chamber. (S150) The washing tip and the magnetic beads are moved together vertically to wash the magnetic beads so that the non-specific reactants are removed. (S160) Preferably, the vertical movement may be repeated several times. The washing tip and the magnetic beam are moved together to the detection chamber. (S170) The magnetic beam is moved upward using the driving motor 13. (S180) The magnetic beads is detached from the surface of the washing tip and scattered in the detection chamber when the magnetic beam is moved upward.

Hereinafter, the effects of the present invention will be described in detail using the embodiments. However, the scope of the present invention is not limited thereto.

### Embodiment 1 - the experiment of sandwich immunoassay of HCG - the present invention

Using an automated immunoassay ALFIS-3, the washing tip where the magnetic beam is positioned is repeatedly moved vertically upward and downward together in the washing chamber to physically wash the magnetic beads. The detection results after physically washing the magnetic beads away is shown on the right side of Table 1.

### Comparative Example 1 - the experiment of sandwich immunoassay of HCG - the prior art

Using an automated immunoassay ALFIS-3, only the magnetic beam is repeatedly moved vertically upward and downward without moving the washing tip in the washing chamber to repeat scattering and collecting the magnetic beads so that the magnetic beads may be washed away. The detection results after washing the magnetic beads away is shown on the left side of Table 1.

**[Table 1]**

| **HCG** | **1st - Dispersion & Capturing** | | | Mean | CV | **HCG** | **1ₛₜ - No Dispersion** | | | Mean | CV |
|---|---|---|---|---|---|---|---|---|---|---|---|
| Concentration | Test 1 | Test 2 | Test 3 | | | Concentration | Test 1 | Test 2 | Test 3 | | |
| Low | 234 | 224 | 223 | 227 | 3% | Low | 214 | 221 | 220 | 218 | 2% |
| Middle | 774 | 709 | 654 | 712 | 8% | Middle | 725 | 722 | 711 | 719 | 1% |
| High | 4742 | 4547 | 4105 | 4465 | 7% | High | 4370 | 4359 | 3849 | 4193 | 7% |

| **HCG** | **2_{nd} - Dispersion & Capturing** | | | Mean | CV | **HCG** | **2_{nd} - No Dispersion** | | | Mean | CV |
|---|---|---|---|---|---|---|---|---|---|---|---|
| Concentration | Test 1 | Test 2 | Test 3 | | | Concentration | Test 1 | Test 2 | Test 3 | | |
| Low | 232 | 225 | 216 | 224 | 4% | Low | 234 | 222 | 224 | 227 | 3% |
| Middle | 732 | 674 | 645 | 684 | 6% | Middle | 676 | 697 | 685 | 686 | 2% |
| High | 4513 | 4433 | 4066 | 4337 | 5% | High | 4498 | 4118 | 4255 | 4290 | 4% |

| **HCG** | **3_{rd} - Dispersion & Capturing** | | | Mean | CV | **HCG** | **3_{rd} - No Dispersion** | | | Mean | CV |
|---|---|---|---|---|---|---|---|---|---|---|---|
| Concentration | Test 1 | Test 2 | Test 3 | | | Concentration | Test 1 | Test 2 | Test 3 | | |
| Low | 227 | 221 | 226 | 225 | 1% | Low | 229 | 225 | 221 | 225 | 2% |
| Middle | 740 | 690 | 671 | 700 | 5% | Middle | 715 | 686 | 671 | 691 | 3% |
| High | 4262 | 3966 | 4315 | 4181 | 4% | High | 4292 | 4454 | 4304 | 4350 | 2% |

### Embodiment 2 - FT4 (competition assay) - the present invention

Using an automated immunoassay ALFIS-3, the washing tip on which the magnetic beam is positioned is repeatedly moved together vertically upward and downward so that the magnetic beads may be physically washed. The detection results after physically washing the magnetic beads away is shown on the right side of Table 2.

### Comparative Example 2 - FT4 (competition assay) - the prior art

Using an automated immunoassay ALFIS-3, only the magnetic beam is repeatedly moved vertically upward and downward without moving the washing tip in the washing chamber to repeat scattering and collecting the magnetic beads so that the magnetic beads may be washed away. The detection results after washing the magnetic beads away is shown on the left side of Table 2.

**[Table 2]**

| **FT4** | **1ₛₜ - Dispersion & Capturing** | | | Mean | CV | **FT4** | **1ₛₜ - No Dispersion** | | | Mean | CV |
|---|---|---|---|---|---|---|---|---|---|---|---|
| Concentration | Test 1 | Test 2 | Test 3 | | | Concentration | Test 1 | Test 2 | Test 3 | | |
| Low | 2654 | 2731 | 2412 | 2599 | 6% | Low | 2369 | 2490 | 2469 | 2443 | 3% |
| Middle | 1565 | 1404 | 1674 | 1548 | 9% | Middle | 1569 | 1596 | 1716 | 1627 | 5% |
| High | 1253 | 1226 | 1372 | 1284 | 6% | High | 1263 | 1180 | 1299 | 1247 | 5% |

| **FT4** | **2_{nd} - Dispersion & Capturing** | | | Mean | CV | **FT4** | **2_{nd} - No Dispersion** | | | Mean | CV |
|---|---|---|---|---|---|---|---|---|---|---|---|
| Concentration | Test 1 | Test 2 | Test 3 | | | Concentration | Test 1 | Test 2 | Test 3 | | |
| Low | 2377 | 2353 | 2451 | 2394 | 2% | Low | 2287 | 2457 | 2185 | 2310 | 6% |
| Middle | 1430 | 1327 | 1459 | 1405 | 5% | Middle | 1499 | 1474 | 1528 | 1500 | 2% |
| High | 1206 | 1229 | 1219 | 1218 | 1% | High | 1123 | 1099 | 1155 | 1126 | 2% |

| **FT4** | **3_{rd} - Dispersion & Capturing** | | | Mean | CV | **FT4** | **3_{rd} - No Dispersion** | | | Mean | CV |
|---|---|---|---|---|---|---|---|---|---|---|---|
| Concentration | Test 1 | Test 2 | Test 3 | | | Concentration | Test 1 | Test 2 | Test 3 | | |
| Low | 2279 | 2074 | 2434 | 2262 | 8% | Low | 2281 | 2332 | 2459 | 2357 | 4% |
| Middle | 1513 | 1369 | 1612 | 1498 | 8% | Middle | 1546 | 1664 | 1667 | 1626 | 4% |
| High | 1138 | 1100 | 1167 | 1135 | 3% | High | 1220 | 1209 | 1298 | 1242 | 4% |

### Conclusion

Referring to Tables 1 and 2, the CV value (coefficient of variation) is smaller and so the error is smaller when using the washing method of the present invention than when using the conventional washing method. So, it is confirmed that high-sensitivity immunoassay is possible.

## Claims

1. An immunoassay device using magnetic beads, comprising:
a straw arm capable of fixing a washing tip to a lower part and having a hollow penetrating vertically upward and downward inside;
a magnetic beam positioned in a hollow of the straw arm and capable of moving vertically upward and downward;
a movable body to which the straw arm is fixed;
a movable body drive unit for moving the movable body horizontally;
a driving motor for moving the magnetic beam vertically upward and downward; and
a control unit for controlling the movable body drive unit.

2. The device of claim 10, further comprising:
a reaction chamber including magnetic beads to which biological samples and reagents are attached; and
a washing chambers for washing non-specific biological samples which are attached to the magnetic beads.

3. An immunoassay method using magnetic beads, including steps of:
(a) bonding analytes and magnetic beads in biological samples through an antigen-antibody reaction by injecting the biological samples into a reaction chamber that contains the magnetic beads;
(b) moving a washing tip to the first vessel;
(c) positioning a magnetic beam in a hollow portion of the washing tip by lowering the magnetic beam;
(d) collecting the magnetic beads onto a surface of the washing tip where the magnetic beam is positioned;
(e) moving the washing tip which the magnetic beads are attached to a washing chamber;
(f) physically washing non-specific biological samples from a surface of the washing tip to which the magnetic beads are attached in the washing chamber;
(g) moving the washing tip from which the non-specific reactant has been removed to the detection chamber; and
(h) scattering the magnetic beads on the surface of the washing tip in the detection chamber by raising the magnetic beam positioned in the hollow portion of the washing tip.

4. The method of claim 3, wherein the step (f) of physically washing the samples includes a step of:
moving vertically upward and downward the washing tip repeatedly which the magnetic beam is inserted into.
